# EUROPEAN PATENT APPLICATION

(11) **EP 2 270 019 A1**
(43) Date of publication of application: **05.01.2011**
(21) Application number: 09163227.3
(22) Date of filing: 19.06.2009
(51) Int. Cl.: C07F 5/02, C07F 17/00, C07K 5/06

(54) **New synthetic route for the preparation of alpha-amino boronic esters**

(71) Applicant: LEK Pharmaceuticals d.d., 1526 Ljubljana (SI)
(72) Inventor: The designation of the inventor has not yet been filed

(57) **Abstract**

The present invention relates to a process for preparing a compound of formula (VI) R₁CH₂-C^{*}(X)-B(OR₂) (OR₃) (VI) wherein: R₁ is hydrogen, substituted or unsubstituted alkyl, substituted or unsubstituted aryl, or substituted or unsubstituted aralkyl; R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and X is selected from the group consisting of Cl, Br, I, OCOR' and OSO₂R', wherein R' represents substituted or unsubstituted alkyl, substituted or unsubstituted aryl or substituted or unsubstituted aralkyl; and ^{*} indicates a chiral center; wherein said process comprises the steps of: (i) providing a compound of formula (V) R₁-CH=CX-B (OR₂) (OR₃) (V), wherein R₁, R₂, R₃ and X are as defined above; and (ii) converting said compound of formula (V) to compound of formula (VI) by hydrogenation, and (iii) optionally applying enantiomeric resolution in order to obtain enantiomerically pure compound of formula (VI).

## Description

### Field of the Invention

The present invention relates in general to the field of organic chemistry and in particular to the preparation of α-amino boronic esters. These compounds can be used as intermediates in the synthesis of boronic acid and ester compounds such as *N*-terminal peptidyl boronic acid derivatives, for example *N-*(pyrazin-2-yl)carbonyl-L-phenylalanine-L-leucine boronic acid, *i*.*e*. bortezomib.

### Background of the Invention

Amino boronic acids - amino acids wherein terminal carboxylic groups are replaced by boronic B(OH)₂ groups - are important pharmacoisosters of amino acids in various therapeutically promising molecules, mainly for treatment of cancer. For instance, talabostat contains proline boronic acid, bortezomib contains leucine boronic acid. Bortezomib, chemically *N*-(pyrazin-2-yl)carbonyl-L-phenylalanine-L-leucine boronic acid, is an important proteasome inhibitor and has been clinically approved for use in treating mantle cell lymphoma and multiple myeloma. Recently, many novel molecules containing amino boronic acids, especially leucine boronic acid, have been prepared and biologically tested as described in WO2009/006473 A2.

The synthesis of bortezomib and other amino boronic acid and ester compounds is disclosed in EP0788360 B1, international patent application WO2005/097809 A2, international patent application WO2009/004350 A1, and international patent application W02009/036281 A2.

EP0788360 B1 describes a general process for preparation of amino boronic acid and ester compounds using (1*S*, 2*S*, 3*R*, 5*S*)-Pinanediol leucine boronate and an amino acid or its derivative as starting materials. As coupling agents 1-Ethyl-3-(3-dimethylamino-propyl)carbodiimide hydrochloride (EDC), benzotriazol-1-yloxytris (dimethylamino) phosphonium hexafluorophosphate (BOP reagent), or *O*-(1H-benzotriazol-1-yl)-*N,N,N',N*'-tetramethyluronium tetrafluoroborate (TBTU) were employed.

A synthetic process suitable for a large scale production of amino boronic acid and ester compounds is described in WO2005/097809 A2. The synthesis involves a boronate complex, which is contacted with a Lewis acid under conditions that afford the boronic ester compounds.

WO2009/004350 A1 discloses a high yield synthesis of bortezomib and intermediates for the synthesis thereof. The procedure includes the use of a very high percentage of tetrahydrofuran in the halogenation of the starting compound (S)-Pinanediol 2-methylpropane-1-boronate.

WO2009/036281 A2 describes processes for the preparation of substantially pure bortezomib and intermediates thereof. Processes for the preparation of crystalline forms of bortezomib as well as a storage system for bortezomib are also disclosed in said patent application.

In international patent application WO2005/097809 A2, in J. Bio/. Chem. 1984, 259, 15106-15114 and in J. Am. Chem. Soc. 1981, 103, 5241-5242 a route for the preparation of α-amino boronic esters, which is known to the person skilled in the art known as the Matteson's synthetic route, is described. Homologation of boronic esters with (dichloromethyl)lithium to form α-chloro boronic esters has been shown to be efficient and result in good chiral selectivity if pinanediol was used as the chiral directing group. The use of the Lewis acid (ZnCl₂) as a catalyst and chloride ion scavenger for the rearrangement of the borate intermediate improved the diastereomeric ratio in the α-chloro boronic ester product. α-Chloro boronic esters have been converted to silylated α-amino boronic esters by lithiumhexamethyldisilazane (LiHMDS), which have been desilylated and protonated *in situ* to the α-amino boronic esters.

An approach for the synthesis of diverse α-amino boronic esters by the highly diastereoselective copper-catalyzed addition of bis(pinacolato)diboron to *N*-*tert*-butane sulfinyl aldimines has been reviewed in the J. Am. Chem. Soc. 2008, 130, 6910-6911.

Transformation of 1,1-dihalogenoalkenes to corresponding alkynes and subsequent synthesis of 1-alkynylboranes have been described in Tetrahedron Letters 1972, 13, 3769-3772 and Tetrahedron Letters 1988, 29, 2631-2634.

J. Am. Chem. Soc. 1994, 116, 10302-10303 describes a process for preparing α-substituted 1-alkenyldioxaborolanes starting from 1-alkynyldioxaborolanes by hydrozirconation followed by substitution such as halogenation or carbonylation. The following α-substituted 1-alkenyldioxaborolanes are disclosed in this reference: (*E*)-2-(1-chloro-3,3-dimethylbut-1-enyl)-4,4,5,5-tetramethyl-1,3,2-dioxaborolane, (*E*)-2-(1-bromo-3,3-dimethylbut-1-enyl)-4,4,5,5-tetramethyl-1,3,2-dioxaborolane, (*E*)-2-(1-iodo-3,3-dimethylbut-1-enyl)-4,4,5,5-tetramethyl-1,3,2-dioxaborolane, (*E*)-2,2,6,6-tetramethyl-4-(4,4,5,5-tetramethyl-1,3,2-dioxaborolan-2-yl)hept-4-en-3-one, (*E*)-4,4-dimethyl-1-phenyl-2-(4,4,5,5-tetramethyl-1,3,2-dioxaborolan-2-yl)pent-2-en-1-one, (*E*)-2-(4,4-dimethylpent-2-en-2-yl)-4,4,5,5-tetramethyl-1,3,2-dioxaborolane zirconocene and (*E*)-2-(hept-2-en-2-yl)-4,4,5,5-tetramethyl-1,3,2-dioxaborolane zirconocene.

There is a need in the art for new intermediate compounds and efficient processes for the preparation of α-substituted boronic esters.

### Summary of the Invention

The present invention provides the following items including main aspects and preferred embodiments, which respectively alone and in combination particularly contribute to solving the above object and eventually provide additional advantages:
(1) A process for preparing a compound of formula VI 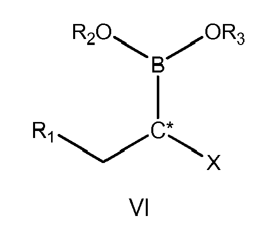 , wherein:
   R₁ is hydrogen, substituted or unsubstituted alkyl, substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
   X is selected from the group consisting of Cl, Br, I, OCOR' and OSO₂R', wherein R' represents substituted or unsubstituted alkyl, substituted or unsubstituted aryl or substituted or unsubstituted aralkyl; and
   * indicates a chiral center;
   wherein said process comprises the steps of:
   (i) providing a compound of formula V 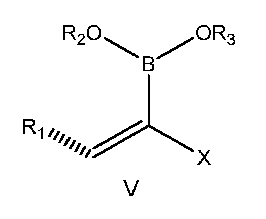
      , wherein R₁, R₂, R₃ and X are as defined above;
      and
   (ii) converting said compound of formula V to compound of formula VI by hydrogenation.
      The term "alkyl" as employed herein includes both straight and branched hydrocarbon chains of up to 12 carbons, preferably 1-8 carbons, such as methyl, ethyl, propyl, *i*-propyl, butyl, *t*-butyl, *i*-butyl, pentyl, hexyl, *i*-hexyl, heptyl, 4,4-dimethylpentyl, octyl, 2,2,4-trimethylpentyl, nonyl, decyl, undecyl and dodecyl, or cyclic hydrocarbons including saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl.
      The term "aryl" as employed herein by itself or as part of another group refers to monocyclic or bicyclic aromatic groups containing from 6 to 12 carbons in the ring portion, preferably 6-10 carbons in the ring portion, such as phenyl or naphthyl.
      The term "arylalkyl" as used herein means that the aforementioned aryl moieties are incorporated into the aforementioned straight or branched alkyl moieties either at one of the proximal or distal ends of the alkyl chain or between the aforementioned alkyl chains. For example, proximal end means for R₁ e.g. adjacent to the double bond of compound of formula V, and for R₂ and R₃ adjacent to the oxygen of compound of formula V and VI, while distal means the terminal end of the arylalkyl moiety.
      The term "substituted" as employed herein includes alkyl, aryl or aralkyl groups as defined above that have one, two or three halogen substituents, or one C₁₋₆ alkyl(C₆₋₁₀)aryl, halo(C₆₋₁₀)aryl, C₃₋₈ cycloalkyl, C₁₋₆ alkyl(C₃₋₈)cycloalkyl, C₂₋₈ alkenyl, C₂₋₈ alkynyl, hydroxy and/or carboxy.
      The term "fused" as used herein means at least two rings connected one to the other with at least one common bond.
      The hatched line as shown in the structural formula of compound of formula V for C-R₁ bonds means that the geometrical configuration is not defined, but compound of formula V may be in form of a mixture of both (*E*) and (*Z*) isomers or in form of pure (*E*) and (*Z*) isomer respectively.
      The procedural concept according to this aspect of the invention involving conversion of compound of formula V to compound of formula VI provides for an industrially applicable and competitive process, since - in contrast to the Matteson's methodology, which is used in prior art to obtain α-substituted boronic esters - there is no difficulty to control rearrangement step. Furthermore, choosing compound of formula V as the starting material enables the use of inexpensive and readily available prior starting materials to obtain such a compound, while the use of toxic and/or hazardous reagents can be avoided.
      R₂ and R₃ may be selected in view of subsequent procedural steps. For example, in case R₂ and R₃ are used as protecting group(s) only, achiral R₂ and R₃ may be used which can be introduced by readily available and/or inexpensive reagents. On the other hand, if R₂ and R₃ represent an integral part of the compound to be produced, or if R₂ and R₃ act as directing group(s), then suitable chiral group(s) may be selected for R₂ and R₃.
(2) The process according to item (1), wherein R₁ is selected from a group consisting of hydrogen, substituted or unsubstituted linear C₁-C₅-alkyl, substituted or unsubstituted branched C₁-C₅-alkyl and substituted or unsubstituted C₃-C₈cycloalkyl, wherein R₂ and R₃ are selected from a group consisting of linear substituted or unsubstituted C₁-C₅-alkyl, substituted or unsubstituted branched C₁-C₅-alkyl, or R₂ and R₃ form a part of a 5- to 10-membered fused or unfused ring, optionally a chiral 5- to 10-membered fused or unfused ring; preferably R₁ is unsubstituted linear or branched C₁-C₅-alkyl, and/or R₂ and R₃ form a part of a 5-membered ring; more preferably, R₁ is isopropyl and/or R₂ and R₃ cooperatively form a part of a 5-membered ring representing 4,4,5,5-tetramethyl-[1,3,2] dioxaborolane.
(3) The process according to item (1) or (2), wherein X is a halogen selected from the group consisting of Cl, Br and I.
(4) The process according to any one of the preceding items, wherein said hydrogenation is conducted in the presence of a catalyst, preferably said catalyst is a catalyst for homogeneous catalysis.
(5) The process according to item (4), wherein the catalyst is selected from complexes comprising transition metal(s).
   In a complex comprising transition metal(s), i.e. catalyst, the transition metal is preferably complexed with at least one ligand containing electron-rich species such as various double bonded compounds and/or free electron pair containing O, N, S, or P species. More preferably, the transition metal catalyst has chirality in the ligand and/or at the transition metal atom, or the transition metal complex having chirality is formed in situ by using an achiral procatalyst comprising the transition metal together with a cocatalyst having chirality, such as a chiral ligand. Even more preferably, the aforementioned components having chirality are in enantiontiopure or diastereomerically pure form. In particular, at least one of said ligands has chirality, wherein said ligand(s) is/are in enantiopure or diasteriomerically pure form. Such ligands for instance may include, but are not limited to, (*R*)-(+)-2,2',6,6'-tetramethoxy4,4'-bis(diphenylphosphino)-3,3'-bipyridine (i.e. (*R*)-P-Phos); (*S*)-2,2',6,6'-tetramethoxy-bis [di(3,5-dimethylphenyl) phosphino]-3,3'-bipyridine (i.e. (*S*)-Xyl-P-Phos); (*R*)-4,12-bis(diphenylphosphino)-[2.2]-paracyclophane (i.e. (*R*)-phane-Phos); 1-(*S*)-N-methyl-N-(diphenylphosphino)-1-[(*R*)-(diphenylphosphino)-ferrocenyl]ethylamine (i.e. (*S*)-MeBoPhos); (*R*)-2-(1-naphthyl)-8-diphenylphosphino-1-(*S*)-3,5-dioxa-4-phosphacyclohepta[2,1-1;3,4-a'] di-naphthalen-4-yl)-1,2-dihydroquinoline toluene aduct (i.e. (*S*a,*R*c)-(1-Nph)-Quinaphos); (*S*)-(+)-4,12-bis[di(3,5-dimethylphenyl)phosphino]-[2.2]paracyclophane (i.e. (*S*)-XylPhanePhos); (*R*)-2,2'-bis (diphenylphosphinoamino)-5,5',6,6',7,7',8,8'-octahydro-1,1'-binaphthyl (i.e. (*R*)-H8-Binam-P). Preferably, the complex comprising transition metal(s) is used at a molar substrate to catalyst ratio in the range of 25:1 to 100:1, more preferably at a molar substrate to catalyst ratio of 50:1.
(6) The process according to item (5), wherein the transition metal comprised in the complex is selected from the group consisting of Cu, Co, Ni, Rh, Ru, Pd and Ir, preferably Rh, Ru, Pd and Ir, more preferably Ru and Ir, and in particular Ir.
(7) The process according to item (5) or (6), wherein the catalyst is (1,5-cyclooctadiene)(pyridine)(tricyclohexylphosphine)iridium(I) hexafluorophosphate.
(8) The process according to any one of the preceding items, wherein hydrogenation is carried out at a temperature from about 10 °C to 80 °C, preferably at about 45 °C to 55 °C, more preferably about 50°C.
   The term "about" as used herein means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10 %.
(9) The process according to any one of the preceding items, wherein hydrogenation is carried out at a pressure of hydrogen of about 10 bar; and/or
   wherein the reaction time is about 1 to 20 days, preferably about 10 days.
(10) The process according to any one of items (3) to (9), wherein dehalogenation is essentially avoided during hydrogenation, preferably dehalogenation occurs in less than 10 molar %, more preferably in less than 5 molar %, most preferably in less than 3 molar %, in particular in less than molar 1% relative to the molar amount of compound of formula VI.
   The term "dehalogenation" as used herein means a reaction wherein a halogen is removed from a compound and replaced by hydrogen.
   During a hydrogenation process halogen attached to a double bond of alkenes is usually submitted to dehalogenation. According to this beneficial embodiment of the invention, it has been surprisingly found that substituted a-halogenovinyl boronic esters of formula V can be hydrogenated to arrive at α-halogeno boronic esters of formula VI without a substantial risk of dehalogenation. By further choosing a suitable catalyst which provides for mild reaction conditions, dehalogenation of compounds of formula V can be essentially avoided.
(11) The process according to any one of the preceding items, wherein enantiomerically pure compound of formula VI is obtained by enantiomeric resolution applied subsequent to step (ii).
   "Enantiomeric resolution" as employed herein means separating enantiomers by means known in the art such as chiral column chromatography or by crystallization of diastereomeric salts.
(12) The process according to any one of items (1) to (11), wherein the compound of formula VI 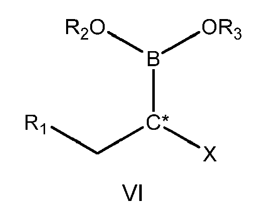
   , wherein R₁, R₂ and R₃ are as defined above, and X is selected from the group consisting of Cl, Br, I, OCOR' and OSO₂R',
   is further converted to a compound of formula VI' 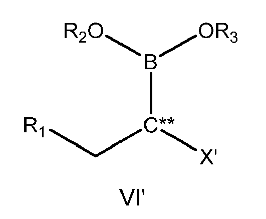
   , wherein R₁, R₂ and R₃ are as defined above; and
   X' is selected from the group consisting of Cl, Br, I, OCOR' and OSO₂R', and X' is not the same as X,
   wherein a chiral center ** has the opposite stereochemical configuration of the chiral center *.
   by applying nucleophilic substitution reaction of type S_{N}2.
   According to this preferred embodiment of the invention, the stereochemical configuration of the chiral center of compound of formula VI can be converted into a chiral center having the opposite stereochemical configuration. This provides for conversion of an "undesired" stereoisomer into a "desired" stereoisomer. The term "desired stereoisomer" as used herein means a compound of formula VI having the desired an stereochemical configuration at the α-carbon atom for further synthesis such as production of a N-terminal peptidyl boronic acid derivative of formula X or a bortezomib derivative. The term "undesired" stereoisomer as used herein means a compound of formula VI having an stereochemical configuration at the α-carbon atom which is opposite to the configuration of the desired stereoisomer. For example, the (*S*)-enantiomer may represent the undesired stereoisomer and therefore be converted into the desired (*R*)-enantiomer respectively. "Undesired" stereoisomer may e.g. be obtained from enantiomeric resolution.
   Furthermore, this embodiment provides for a flexible application of "desired" compound of formula VI in a multitude of different subsequent syntheses, since the stereochemical configuration at the chiral center can subsequently be suitably selected in case the pathway of synthesis or even the target product of the synthetic pathway is changed.
(13) The process according to any one of the preceding items, wherein the compound of formula V 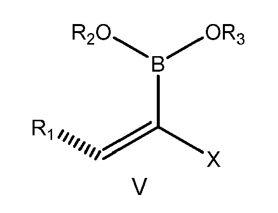
   , wherein R₁ is hydrogen, substituted or unsubstituted alkyl substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
   X is selected from the group consisting of Cl, Br, I, OCOR' and OSO₂R', wherein R' represents substituted or unsubstituted alkyl, substituted or unsubstituted aryl or substituted or unsubstituted aralkyl,
   is prepared from a compound of formula III 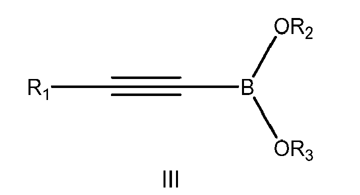
   , wherein R₁, R₂ and R₃ are as defined above,
   by hydrozirconation converting compound of formula III into compound of formula IV 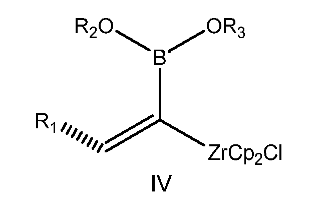
   , wherein the ZrCp₂Cl moiety of compound of formula IV is substituted by X as defined above.
   The hatched line as shown in the structural formula of compound of formula IV for C-R₁ bonds means that the geometrical configuration is not defined, but compound of formula IV may be in form of a mixture of both (*E*) and (*Z*) isomers or in form of pure (*E*) and (*Z*) isomer respectively.
   The term "hydrozirconation" as employed herein means a hydro-metallo-addition of alkenes and alkynes to give organometallic compounds, by using zirconium containing reagent, Cp₂ZrHCl, also known as the Schwartz's reagent.
   For the terms "alkyl", "aryl", "aralkyl" and "substituted", reference is made to the corresponding definitions under item (1).
(14) The process according to item (13), wherein ZrCp₂Cl moiety of compound of formula IV is substituted by halogen selected from Cl, Br, I, preferably Cl, by means of halogenation, preferably by halogenation *in situ.*
   The term "halogenation" as employed herein means a reaction of substitution in which organometallic compounds comprising Schwartz's intermediate react with halogens to give alkenyl or aryl halides.
(15) The process according to item (14), wherein halogenation is carried out by a reactant selected from the group consisting of Cl₂ Br₂, I₂, *N*-chlorosuccinimide, *N*-bromosuccinimide and *N-*iodosuccinimide, preferably *N*-chlorosuccinimide, *N*-bromosuccinimide and *N*-iodosuccinimide, more preferably *N*-chlorosuccinimide; and/or
   wherein the solvent is selected from the group consisting of THF, CH₂Cl₂, Et₂O, MTBE, *i*-Pr₂O, MeTHF, preferably CH₂Cl₂ and THF, more preferably THF.
(16) The process according to items (13) to (15), wherein the halogen is optionally further converted to a moiety selected from the group consisting of OCOR' and OSO₂R', wherein R' represents substituted or unsubstituted alkyl, substituted or unsubstituted aryl or substituted or unsubstituted aralkyl.
(17) A process for preparing a compound of formula III 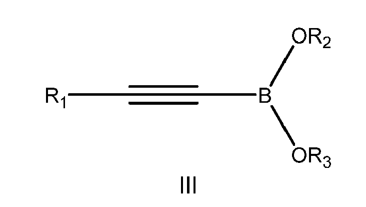
   , wherein R₁ is hydrogen, substituted or unsubstituted alkyl substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
   wherein said process comprises the steps of:
   (i) providing a compound of formula I 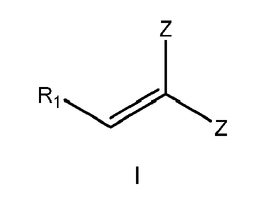 , wherein R₁ is hydrogen, substituted or unsubstituted alkyl substituted or unsubstituted aryl, or substituted or unsubstituted aralkyl;
      Z is selected from the group consisting of Cl, Br and I, preferably Cl,
   (ii) adding a strong organometallic base to a solution of a compound of formula I in order to convert compound of formula I to compound of formula II 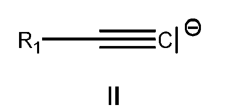 , wherein R₁ is defined as above,
   (iii) reacting the compound of formula II with a compound of formula IX 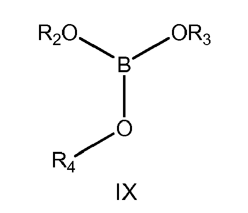 , wherein R₂ and R₃ are defined as above,
      and R₄ represents substituted or unsubstituted alkyl, substituted or unsubstituted aryl, or substituted or unsubstituted aralkyl, wherein R₄ is same or different group as R₂ and/or R₃,
      in the presence of a solvent and followed by addition of an acid,
      wherein steps (i), (ii) and (iii) are performed as a one pot reaction without isolation of compound of formula II or its respective acetylene derivative R₁C=CH.
   For the terms "alkyl", "aryl", "aralkyl" and "substituted", reference is made to the corresponding definitions under item (1).
   According to this aspect of the invention, a process for preparing 1-alkynylboronates from dihalogenoalkenes is provided, wherein an acetylide carbanion of formula II is formed by adding a strong organometallic base to a compound of formula I. The term "strong organometallic base" as employed herein means an organometallic base having a pKa value of at least 35. The acetylide carbanion of formula II is reacted with compound of formula IX without isolation of the respective acetylene derivative R₁C=CH. Acetylene derivatives R₁C=CH derived from compounds of formula I are normally very volatile, that is their boiling point is close to or within the range of ambient temperatures. Thus, this procedural concept provides for a save and improved operability of the process, since no isolation of said relatively volatile acetylene derivative is necessary. Furthermore, the combination of directly converting compound of formula I to compound of formula II and *in situ* reaction of said compound of formula II with compound of formula IX provides for an advantageous one pot reaction wherein yields are improved since there is no laborious isolation of intermediate products resulting in yield losses.
   Furthermore, this aspect of the invention provides valuable starting materials for the preparation of compounds of formula VI by the process according to items (1) to (12), wherein compound of formula V is preferably obtained by the process according to items (13) to (16).
   Preferably, R₂, R₃ and R₄ are the same or R₂, R₃ cooperatively form a part of a 5- to 10-membered fused or unfused ring, while R₄ represents a group different from R₂, R₃, more preferably R₂, R₃ and R₄ are the same representing substituted or unsubstituted alkyl group, or R₂, R₃ cooperatively form a part of a 5- to 8-membered fused or unfused ring while R₄ represents a substituted or unsubstituted alkyl group, in particular R₂, R₃ and R₄ are the same representing unsubstituted alkyl group, or R₂, R₃ cooperatively form a part of a 5- to 6-membered ring while R₄ represents an unsubstituted alkyl group
(18) The process according to any one of items (13) to (16), wherein the compound of formula III 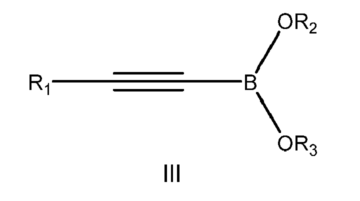 , wherein R₁ is hydrogen, substituted or unsubstituted alkyl substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
   is prepared from a compound of formula II 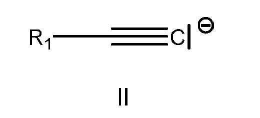 , wherein R₁, is as defined above,
   by reacting the compound of formula II with a compound of formula IX 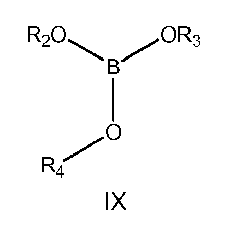 , wherein R₂ and R₃ are defined as above,
   and R₄ represents substituted or unsubstituted alkyl, substituted or unsubstituted aryl, or substituted or unsubstituted aralkyl, wherein R₄ is same or different group as R₂ and/or R₃,
   in the presence of a solvent and followed by addition of an acid.
   For the terms "alkyl", "aryl", "aralkyl" and "substituted", reference is made to the corresponding definitions under item (1). For the preferred groups represented by R₂, R₃, and R₄, reference is made to the corresponding definitions under item (17).
(19) The process according to item (17) or (18), wherein a solvent is used which is selected from the group consisting of diethylether, *i*-Pr₂O, MTBE, MeTHF and THF, preferably THF.
(20) The process according to any one of items (17) to (19), wherein reacting the compound of formula II with a compound of formula IX is followed by addition of an acid, preferably an inorganic acid, in particular HCl.
(21) The process according to any one of items (17) to (20), wherein the compound of formula II is prepared from a compound of formula I 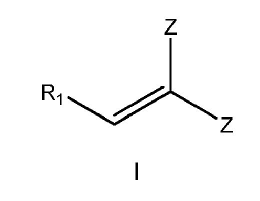 , wherein R₁ is hydrogen, substituted or unsubstituted alkyl substituted or unsubstituted aryl, or substituted or unsubstituted aralkyl;
   Z is selected from the group consisting of Cl, Br and I, preferably Cl;
   by adding a strong organometallic base, to a solution of a compound of formula I.
   In this way, readily available and inexpensive starting materials having the formula I can be used for the synthesis of α-substituted boronic esters of formula VI. α-substituted boronic esters prepared by the Matteson's synthetic route used in prior art are prepared from alkyl or aryl boronic acids from which only few are commercially available, and the esterification reaction of the boronic acid with the respective alcohol needs an additional reaction step. In contrast to that, the present process starts from compounds of formula I, which are obtained from inexpensive and commercially available aldehydes of formula R₁CHO, as e.g. described in Tetrahedron Letters 2000, 41, 4007-4009.
(22) The process according to item (17) or (21), wherein the strong organometallic base is selected from the group consisting of metal amides and alkyl lithiums, preferably sodium amide, potassium amide, lithium amide, lithium diisopropylamide, methyllithium, butyllithium, hexyllithium and phenyllithium, more preferably butyllithium (*n*-BuLi) or methyllithium, even more preferably *n-*BuLi, in particular *n*-BuLi.
(23) The process according to any one of items (17) to (22), wherein R₂ and/or R₃ of compound of formula IX is/are chiral, preferably a borolane ring formed by R₂ and R₃ of compound of formula IX is chiral, more preferably said chiral compounds of formula IX are enantiomerically or diastereomerically pure.
   According to this preferred embodiment, R₂ and R₃ is suitably selected in view of subsequent reaction steps, for example in cases wherein R₂ and R₃ represent an integral part of the final compound to be produced, or e.g. if R₂ and R₃ act as directing group(s) in subsequent reaction steps.
(24) The process according to any one of items (1) to (12), wherein the compound of formula VI 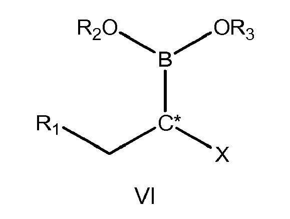 , wherein R₁, R₂ and R₃ are defined as above, and X is selected from the group consisting of Cl, Br, I, OCOR' and OSO₂R', and * indicates a chiral center;
   is further converted to a compound of formula VIII 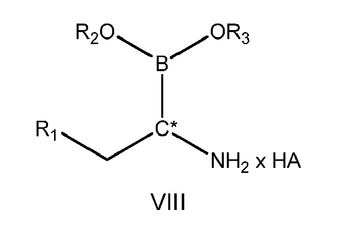 , wherein R₁, R₂ and R₃ are as defined above; and
   A is an anion selected from the group of anions consisting of Cl⁻, Br⁻, HSO4⁻, CH₃COO⁻, CF₃COO⁻ and R'SO₃⁻, wherein R' represents alkyl or aryl; and
   * indicates a chiral center;
   by a process comprising the steps of:
   (i) converting the compound of formula VI to compound of formula VII 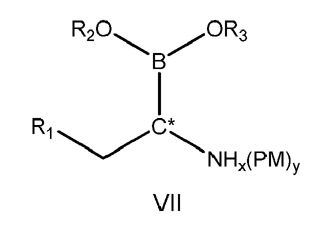 , wherein R₁, R₂ and R₃ are as defined above, and
      PM is an amino group protecting moiety, wherein PM is selected from the group consisting of *tert-*butanesulfinyl (SO-*t*-Bu), tosyl (Ts), *p*-nitrobenzenesulfonyl (Ns), carbobenzyloxy (Cbz), *t-*butyloxycarbonyl (Boc), benzyl (Bn), *p*-methoxybenzyl (MPM), dimethoxybenzyl (Dmb), *p-*hydroxybenzyl (HBn), 9-phenylfluoren-9-yl (Pf), fluorenyl (Flu), diphenylmethyl (DPM), ferrocenylmethyl (Fcm) and 4-Methyltrityl (Mtt), and x = 1 and y = 1, or PM is SiR"₃, wherein R" represents alkyl and x = 0 and y = 2; and
      * indicates a chiral center;
      by substituting X of the compound of formula VI with a protected amino group,
      and
   (ii) subjecting the compound of formula VII to a cleavage of protecting groups to yield the compound of formula VIII, and
   (iii) optionally applying enantiomeric resolution in order to obtain enantiomerically pure compound of formula VIII.
   According to this beneficial embodiment of the invention, α-substituted boronic esters of formula VI are used as intermediates in the synthesis of α-amino boronic esters having a protected amino group.
   A term "cleavage of protecting groups" as employed herein means a reaction in which an amino group protecting moiety is cleaved, e.g. hydrogenation or hydrolysis.
(25) The process according to item (24), wherein the amino group protecting moiety PM is selected from the group consisting of SO-*t*-Bu, Si(CH₃)₃ and Si(CH₂CH₃)₃.
(26) The process according to item (24) or (25), wherein amino group protecting moiety PM is introduced by using salts of silyl amides as reactant, preferably ((CH₃)₃Si)₂NNa (NaHMDS) and ((C₂H₅)₃Si)₂NNa.
(27) The process according to item (24) or (25), wherein A is selected from the group consisting of Cl⁻ and CF₃COO⁻, preferably A is CI⁻; and/or
   X is Cl; and/or amino group protecting moiety PM is Si(CH₃)₃.
(28) The process according to items (24) to (27), wherein the compound of formula VIII in racemic mixture can be further separated to optically pure (*R*)-enantiomer or (*S*)-enantiomer by enantiomeric resolution by crystallization with chiral acids, or by chiral chromatography.
(29) A compound of formula IV 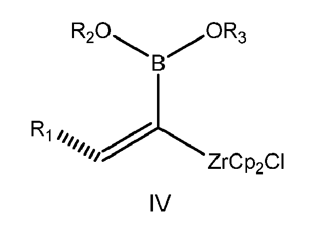 , wherein
   R₁ is hydrogen, substituted or unsubstituted alkyl substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
   with the proviso that R₁ does not denote *t*-Bu or *n*-Bu.
   For the terms "alkyl", "aryl", "aralkyl" and "substituted", reference is made to the corresponding definitions under item (1).
(30) A compound of formula V 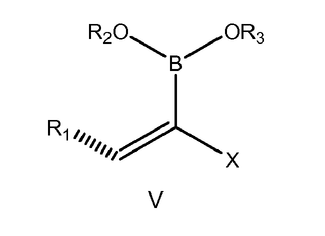 , wherein
   R₁ is hydrogen, substituted or unsubstituted alkyl, substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and X is selected from the group consisting of Cl, Br, I, OCOR' and OSO₂R', wherein R' represents alkyl or aryl;
   with the proviso that if X is Cl, Br, I, then R₁ does not denote *t*-Bu.
   For the terms "alkyl", "aryl", "aralkyl" and "substituted", reference is made to the corresponding definitions under item (1).
(31) A compound of formula IVa 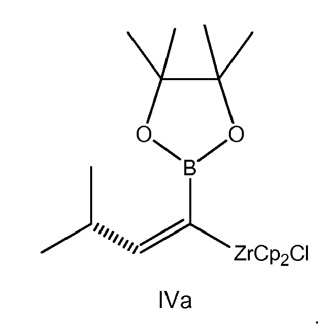
(32) A compound of formula Va 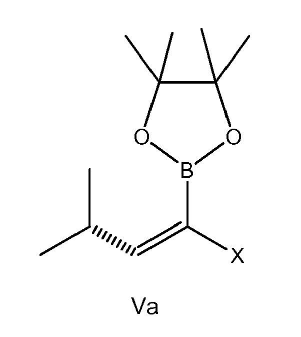 , wherein X is selected from the group consisting of Cl, Br, I, OCOR' and OSO₂R', wherein R' represents substituted or unsubstituted alkyl, substituted or unsubstituted aryl or substituted or unsubstituted aralkyl.
(33) A process for producing a N-terminal peptidyl boronic acid derivative of formula X 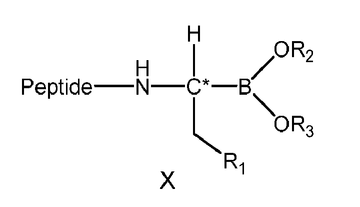 , wherein R₁ is hydrogen, substituted or unsubstituted alkyl, substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
   peptide comprises 1-6 amino acids coupled to each other by peptide bonds with optionally acylated terminal amino group; and
   wherein the chiral center * is in its (*R*) or (*S*) configuration;
   or free acid or ester or anhydride or salt thereof comprising the steps of:
   (i) providing a compound of formula VI prepared by a process according to any one of items (1) to (12),
   (ii) converting said compound of formula VI to compound of formula VIII by a process according to any one of items (24) to (28), and
   (iii) converting said compound of formula VIII to N-terminal peptidyl boronic acid derivative of formula X, preferably free acid or ester or anhydride or salt thereof.
   The term "free acid or ester or anhydride or salt thereof" as used herein means that the boronic acid moiety of the *N*-terminal peptidyl boronic acid derivative of formula X is in form of a free acid or ester or anhydride or salt.
(34) A process for producing bortezomib (*N*-(pyrazin-2-yl)carbonyl-L-phenylalanine-L-leucine boronic acid) or ester or anhydride or salt thereof, comprising the steps of:
   (i) providing a compound of formula VI prepared by a process according to any one of items (1) to (12),
   (ii) converting said compound of formula VI to compound of formula VIII by a process according to any one of items (24) to (28), and
   (iii) converting said compound of formula VIII to bortezomib or ester or anhydride or salt thereof.
   The term "ester or anhydride or salt thereof" as used herein means that the boronic acid moiety of bortezomib (*N*-(pyrazin-2-yl)carbonyl-L-phenylalanine-L-leucine boronic acid) is in form of an ester or anhydride or salt.
   According to this preferred embodiment, an advantageous process for producing *N*-terminal peptidyl boronic acid derivatives of formula X, for example bortezomib is provided, wherein compound of formula VI as the starting material enables the use of inexpensive and readily available starting materials, while the use of toxic and/or hazardous reagents can be avoided.
(35) A process for producing a pharmaceutical composition, comprising the steps of:
   (i) preparing *N*-terminal peptidyl boronic acid derivative of formula X or free acid or ester or anhydride or salt thereof according to item (33), and
   (ii) mixing said compound of formula X with at least one excipient.
(36) A process for producing a pharmaceutical composition, comprising the steps of:
   (i) preparing bortezomib (*N*-(pyrazin-2-yl)carbonyl-L-phenylalanine-L-leucine boronic acid) or anhydride or salt thereof according to item (34), and
   (ii) mixing said bortezomib with at least one excipient.
(37) Use of a compound of formula V 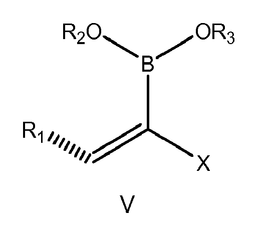 , wherein
   R₁ is hydrogen, substituted or unsubstituted alkyl substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and X is selected from the group consisting of Cl, Br, I, OCOR' and OSO₂R', wherein R' represents alkyl or aryl;
   in the process for preparation of compound of formula VIII 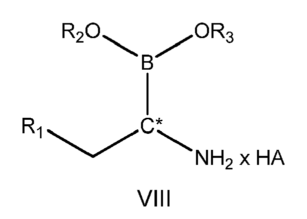 , wherein R₁, R₂ and R₃ are as defined above; and
   A is an anion selected from the group of anions consisting of Cl⁻, Br⁻, HSO₄⁻, CH₃COO⁻, CF₃COO⁻ and R'SO₃⁻, wherein R' represents alkyl or aryl; and
   * indicates a chiral center; or free amine thereof,
   and/or N-terminal peptidyl boronic acid derivative of formula X or free acid or ester or anhydride or a pharmaceutically acceptable salt thereof, preferably bortezomib or ester or anhydride or a pharmaceutically acceptable salt thereof.
   For the terms "alkyl", "aryl", "aralkyl" and "substituted", reference is made to the corresponding definitions under item (1).
(38) Use of a compound of formula IVa 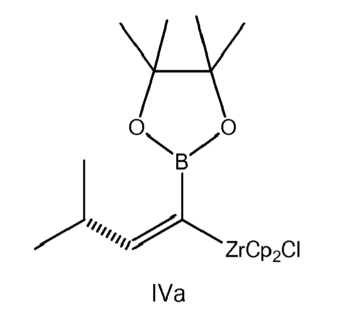 in the process for preparation of compound of formula VIIIa

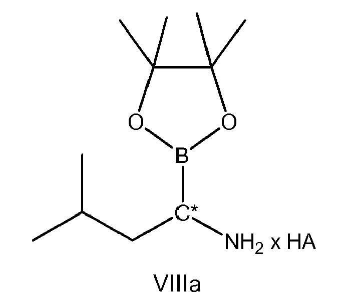

   wherein A is an anion selected from the group of anions consisting of Cl⁻, Br⁻, HSO₄, CH₃COO⁻, CF₃COO⁻ and R'SSO₃⁻, wherein R' represents alkyl or aryl; * indicates a chiral center; or free amine thereof,
   and/or *N-*terminal peptidyl boronic acid derivative of formula X or free acid or ester or anhydride or a pharmaceutically acceptable salt thereof, preferably bortezomib or ester or anhydride or a pharmaceutically acceptable salt thereof.
(39) Use of a compound of formula Va 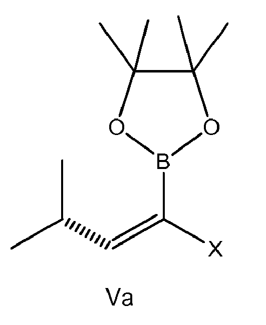 , wherein X is selected from the group consisting of Cl, Br, I, OCOR' and OSSO₂R', in the process for preparation of compound of formula VIIIa 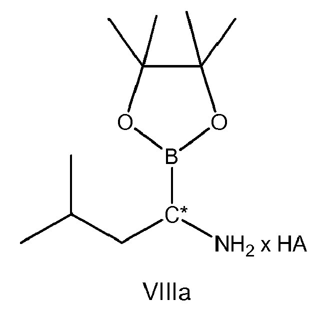 , wherein A is an anion selected from the group of anions consisting of Cl⁻, Br⁻, HSO₄, CH₃COO⁻,
   CF₃COO⁻ and R'SO₃⁻, wherein R' represents alkyl or aryl;
   * indicates a chiral center; or free amine thereof,
   and/or N-terminal peptidyl boronic acid derivative of formula X or free acid or ester or anhydride or a pharmaceutically acceptable salt thereof, preferably bortezomib or ester or anhydride or a pharmaceutically acceptable salt thereof.
(40) Use of an iridium catalyst for hydrogenation of compounds of formula XI where dehalogenation occurs in less than 10 molar %, more preferably in less than 5 molar %, most preferably in less than 3 molar %, in particular in less than molar 1% relative to the molar amount of hydrogenated, non-dehalogenated product obtained from the compound of formula XI: 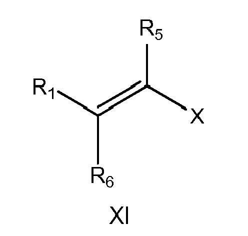 , wherein
   R₁ and R₆ is hydrogen, substituted or unsubstituted alkyl substituted or unsubstituted aryl, or substituted or unsubstituted aralkyl;
   R₅ is OR', NR'R", SR', X", B(OR')₂, in which X" is selected from F, Cl, Br, I, OCOR', OSO₂R' and R' is substituted or unsubstituted alkyl substituted or unsubstituted aryl, or substituted or unsubstituted aralkyl
   X is selected from Cl, Br, I,
   According to this aspect of the invention, a process for the use of an iridium catalyst for hydrogenation of compounds of formula XI is provided, where dehalogenation, which is a common side reaction of hydrogenation of halogen alkanes, e.g. chloroalkenes, is surprisingly reduced to an industrially applicable level in the preparation of haloalkanes from haloalkenes.
(41) The use according to item (40), wherein R₅ is B(OR')₂.
(42) Use of an iridium catalyst for hydrogenation of compounds of formula XII where dehalogenation occurs in less than 10 molar %, more preferably in less than 5 molar %, most preferably in less than 3 molar %, in particular in less than molar 1% relative to the molar amount of hydrogenated, non-dehalogenated product obtained from the compound of formula XII: 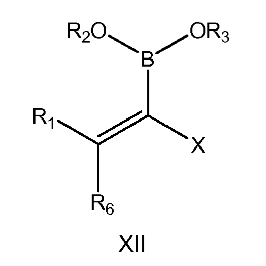 , wherein
   R₁ and R₆ is hydrogen, substituted or unsubstituted alkyl substituted or unsubstituted aryl, or substituted or unsubstituted aralkyl;
   R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
   X is Cl, Br, I.
(43) The use according to item (42), wherein R₁ is selected from a group consisting of hydrogen, substituted or unsubstituted linear C₁-C₅-alkyl, substituted or unsubstituted branched C₁-C₅-alkyl and substituted or unsubstituted C₃-C₈-cycloalkyl, wherein R₂ and R₃ are selected from a group consisting of linear substituted or unsubstituted C₁-C₅-alkyl, substituted or unsubstituted branched C₁-C₅-alkyl, or R₂ and R₃ form a part of a 5- to 10-membered fused or unfused ring, optionally a chiral 5- to 10-membered fused or unfused ring; preferably R₁ is unsubstituted linear or branched C₁-C₅-alkyl, and/or R₂ and R₃ form a part of a 5-membered ring; more preferably, R₁ is isopropyl and/or R₂ and R₃ cooperatively form a part of a 5-membered ring representing 4,4,5,5-tetramethyl-[1,3,2] dioxaborolane.
(44) The use according to item (42) or (43), wherein R₆ is hydrogen.
(45) The use according to any one of items (40) to (44), wherein the iridium catalyst comprises at least one ligand containing electron-rich species such as various double bonded compounds and/or free electron pair containing O, N, S, or P species, preferably the iridium catalyst has chirality in the ligand and/or at the iridium atom, or the iridium complex having chirality is formed in situ by using an achiral iridium procatalyst with a cocatalyst having chirality.
   For the chirality comprised in the catalyst, reference is made to the corresponding definition under item (5).
(46) The use according to any one of items (40) to (45), wherein the iridium catalyst is used at a molar substrate to catalyst ratio in the range of 25:1 to 100:1, more preferably at a molar substrate to catalyst ratio of 50:1.
(47) The use according to any one of items (40) to (46), wherein the iridium catalyst is (1,5-cyclooctadiene)(pyridine)(tricyclohexylphosphine)iridium(I) hexafluorophosphate.

### Detailed Description of the Invention

In the following, the present invention will be described in more detail by preferred embodiments and examples noting, however, that these embodiments, examples are presented for illustrative purposes only and shall not limit the invention in any way.

Reaction Scheme 1 illustrates a preferred embodiment of the process according to the present invention for preparing an α-substituted boronic ester (VI).

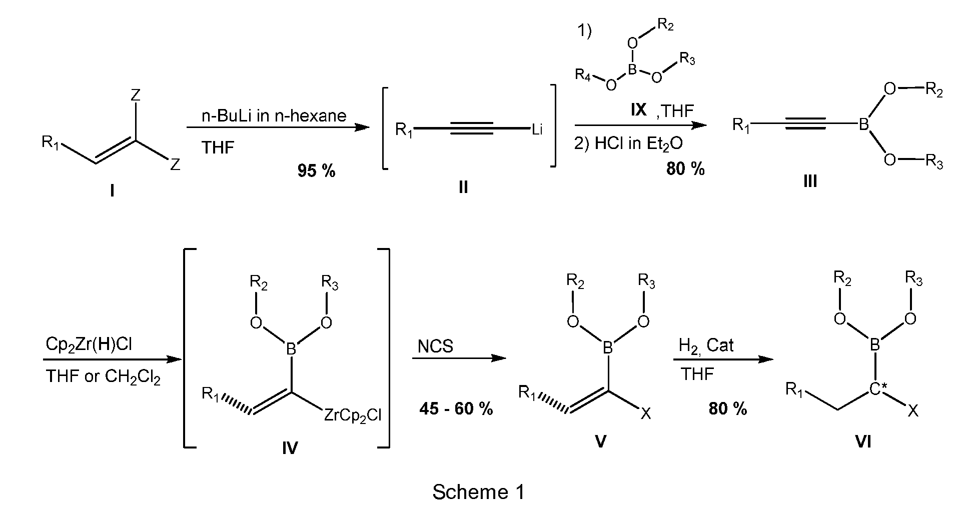

According to the preferred embodiment of Scheme 1 (wherein R₁, R₂, R₃, Z and X are as defined as in the preceding items), a compound of formula III is prepared by contacting a compound of formula I with a strong organometallic base, e.g. an organolithium reagent such as *n*-BuLi in *n*-hexane, to obtain a compound of formula II, which is an acetylide, in this case, since organolithium reagent is used, a lithium acetylide. Then, without isolation of compound of formula II and its respective acetylene derivate R₁C≡CH, the reaction proceeds in the same reaction vessel by adding a compound of formula IX to yield the compound of formula III. The last step of this reaction is carried out by addition of an acid such as anhydrous HCl. Both steps of the reaction, elimination and addition, to compound III are performed in organic solvent, preferably in THF.

The compound of formula I, a starting material of the synthesis presented in Scheme 1, is available; for example, it can be prepared by synthesis routes known to a person skilled in the art, as e.g. described in Tetrahedron Letters 2000, 41, 4007-4009.

Further according to the preferred embodiment illustrated by Scheme 1, a compound of formula V is prepared by subjecting a compound of formula III to hydrozirconation to obtain a compound of formula IV, which is followed by halogenation, preferably *in situ* halogenation. Hydrozirconation as used herein means forming organozirconocenes using Cp₂Zr(H)Cl (also known as Schwartz reagent), a method well known to a person skilled in the art. Halogenation can be achieved for example by adding various halogen reagents which express positive charge on halogen, preferably *N*-halogenosuccinimide (e.g. *N-*chlorosuccinimide, NCS as illustrated in Scheme 1) *in situ* to a compound of formula IV to yield the compound of formula V. The reaction is carried out in organic solvent such as THF or CH₂Cl₂, which is later removed under reduced pressure. The reaction mixture is extracted with *n*-hexane and the residue is purified by chromatography.
Alternatively, halogen introduced subsequent to hydrozirconation as described above, can be optionally further converted to a moiety selected from the group consisting of OCOR' and OSO₂R', wherein R' represents substituted or unsubstituted alkyl, substituted or unsubstituted aryl or substituted or unsubstituted aralkyl, by methods well known from the state of the art. For example, following a protocol of US 4924026 a halogen atom, e.g. Cl (compound of formula V with X = Cl in Scheme 1), can be converted to a moiety OCOMe (compound of formula V with X = OCOMe in Scheme 1).

The hydrohalogenation of alkyne boronates via Schwartz intermediates yields *de novo* formed alkenes, which theoretically exist in two geometrical isomers. Based on the literature data (J. Am. Chem. Soc. 1994, 116, 10302-10303) the (*E*) configuration is supposedly formed predominantly. In the light of the present invention, the determination of the configuration is not necessary, since the β carbon atom is not prochiral in view of the process of our invention.

Further according to the preferred embodiment illustrated by Scheme 1, a compound of formula VI is prepared by hydrogenation/reduction of the compound of formula V. If X in compound of formula V is halogen, then a reduction denotes a homogeneous reduction without dehalogenation. The reaction is performed in an organic solvent, preferably THF, in the presence of a catalyst and is carried out in the autoclave under inert atmosphere.

The catalyst with substantially reduced tendency of dehalogenation is selected from complexes comprising transition metal(s), preferably Cu, Co, Ni, Rh, Ru, Pd, Ir. Preferably, the catalyst is metal-(phosphine)-complex, wherein metal is preferably Cu, Rh, Ru, Pd, Ir, more preferably Ir. Preferably, the transition metal is complexed with at least one organic compound containing electron-rich species such as various double bonded compounds and/or free electron pair containing O, N, S, or P species as a ligand. More preferably, the transition metal catalyst has chirality in the ligand and/or at the transition metal atom, or the transition metal complex having chirality is formed in situ by using an achiral procatalyst comprising the transition metal together with a cocatalyst having chirality, such as a chiral ligand. Even more preferably, the aforementioned components having chirality are in enantiomerically or diastereomerically pure form. In particular, at least one of said ligands has chirality, wherein said ligand(s) is/are in enantiomerically or diasteriomerically pure form. Such ligands for instance may include, but are not limited to, (*R*)-(+)-2,2',6,6'-tetramethoxy-4,4'-bis(diphenylphosphino)-3,3'-bipyridine (i.e. (*R*)-P-Phos); (*S*)-2,2',6,6'-tetramethoxy-bis [di(3,5-dimethylphenyl) phosphino]-3,3'-bipyridine (i.e. (*S*)-Xyl-P-Phos); (*R*)-4,12-bis(diphenylphosphino)-[2.2]-paracyclophane (i.e. (*R*)-Phane-Phos); 1-(*S*)-N-methyl-N-(diphenylphosphino)-1-[(*R*)-(diphenylphosphino)-ferrocenyl]ethylamine (i.e. (*S*)-MeBoPhos); (*R*)-2-(1-naphthyl)-8-diphenylphosphino-1-(*S*)-3,5-dioxa-4-phosphacyclohepta[2,1-1;3,4-a'] di-naphthalen-4-yl)-1,2-dihydroquinoline toluene aduct (i.e. (*S*a,*R*c)-(1-Nph)-quinaphos); (*S*)-(+)-4,12-bis[di(3,5-dimethylphenyl)phosphino]-[2.2]paracyclophane (i.e. (*S*)-XylPhanePhos); (*R*)-2,2'-bis(diphenylphosphinoamino)-5,5',6,6',7,7',8,8'-octahydro-1,1'-binaphthyl (i.e. (*R*)-H8-Binam-P). Furthermore, the complexes comprising transition metal(s) are preferably used at a molar substrate to catalyst ratio in the range of 25:1 to 100:1, more preferably at a molar substrate to catalyst ratio of 50:1. A particularly preferred catalyst is (1,5-cyclooctadiene)(pyridine) (tricyclohexylphosphine)iridium(I) hexafluorophosphate.
A non-limiting list of transition metal catalysts having chiral ligands includes bis(1,5-cyclooctadiene) diiridium(I)dichloride (R)-(+)-2,2',6,6'-tetramethoxy4,4'-bis(diphenylphosphino)-3,3'-bipyridine; bis(1,5-cyclooctadiene)diiridium(I)dichloride (S)-2,2',6,6'-tetramethoxy-bis [di(3,5-dimethylphenyl)phosphino]-3,3'-bipyridine; bis(1,5-cyclooctadiene) dirhodium (I)dichloride (S)-2,2',6,6'-tetramethoxy-bis[di(3,5-dimethylphenyl)phosphino]-3,3'-bipyridine; bis(cycloocta-1,5-diene)rhodium(I) tetrafluoroborate (*R*)-4,12-bis(diphenylphosphino)-[2.2]-paracyclophane; benzeneruthenium(II) dichloride dimer 1-(*S*)-N-methyl-N-(diphenylphosphino)-1-[(*R*)-(diphenylphosphino)-ferrocenyl] ethylamine; bis(2-methylallyl)(1,5-cyclooctadien)ruthenium (II) (*S*)-(+)-4,12-bis[di(3,5-dimethylphenyl)phosphino]-[2.2]paracyclophane;

The compound of formula V, the Ir-catalyst and THF are placed in the autoclave under nitrogen atmosphere. The autoclave is sealed and pressurized/depressurized several times with nitrogen, preferably 3 times with about 6 bar of nitrogen, then several times with hydrogen, preferably 3 times with about 6 bar of hydrogen. The mixture is then stirred for a suitable time period, for example 1 to 20 days, preferably for about 10 days at a temperature from 10 °C to 80 °C, more preferably at about 45 °C to 55 °C under about 10 bar of hydrogen. After this time period the autoclave is cooled to room temperature such as about 20 °C to 25 °C. Then, the autoclave is carefully depressurized and the solution obtained is poured into a suitable vessel, preferably a round bottomed flask. The solvent is removed under reduced pressure and the residue is passed through a short column of silica gel, with suitable eluent, preferably *n-*hexane, to remove the catalyst. Such preferred procedure using an iridium catalyst provides for substantially reduced dehalogenation, preferably dehalogenation occurs in less than 10 molar %, more preferably in less than 5 molar %, most preferably in less than 3 molar %, in particular in less than molar 1% relative to the molar amount of compound of formula VI.

According to another embodiment of the present invention, the racemic mixture of α-(*R*) and α-(*S*) isomers of compound VI obtained in the reaction of hydrogenation of compound V, can be further separated by enantiomeric resolution in order to yield optically pure α-(*R*) or α-(*S*)-enantiomer. Since enantiomers to not differ in their scalar characteristics, enantiomeric resolution needs a chiral environment. A chiral environment for separation may be provided for example by chiral supporters in a chromatographic column or by adding enantiopure acid/base addition salts in order to form diastereomeric salts which can be separated by crystallisation. In a special case wherein the borolane part of compound of formula VI is chiral, compound of formula VI represents a diastereomer. Since diastereomers differ in their scalar characteristics, diastereomeric comounds of formula VI can be separated without providing a chiral environment, e.g by crystallization or chromatographic methods on achiral supporters.

Furthermore, in another embodiment, the leaving group X in the compound of formula VI can be exchanged by X' in order to suitably invert the stereochemical configuration at the α-carbon atom of the compound VI by a process wherein the compound of formula VI 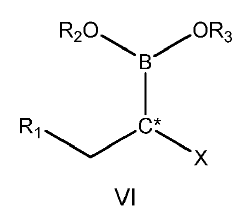 , wherein R₁, R₂ and R₃ are as defined above, and X is selected from the group consisting of Cl, Br, I, OCOR' and OSO₂R',
is further converted to a compound of formula VI' 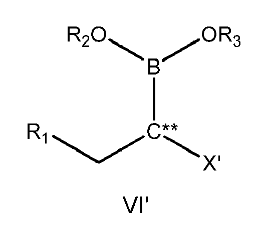 , wherein R₁, R₂ and R₃ are as defined above; and
X' is selected from the group consisting of Cl, Br, I, OCOR' and OSO₂R', and X' is not the same as X,
wherein a chiral center ** has the opposite stereochemical configuration of the chiral center *.
by applying nucleophilic substitution reaction of type S_{N}2.

Said nucleophilic substitution of type S_{N}2 is also known as "Walden inversion" in the art and is rendered possible since the leaving group X is located at a secondary carbon atom which provides for bimolecular S_{N} reaction (S_{N}2) and in turn for inversion of the stereochemical configuration at the α-carbon atom of the compound VI. For example, compound VI wherein X = Cl can be transformed to compound VI' (X = I) by methodology described in EP 0 315 574, or compound VI wherein X = Cl can be converted to compound VI' wherein X = OSO₂Me by methodology described in J. Org. Chem. 1987, 52, 5121-5124. X' is not the same as X, however, X' is selected from the same group as defined for X.

Another preferred aspect of the invention is a process for preparing α-amino boronic ester (VIII) comprising the steps of:
(i) providing a compound of formula VI following the reaction Scheme 1; and
(ii) converting the compound of formula VI to a compound of formula VIII as depicted in Scheme 2.

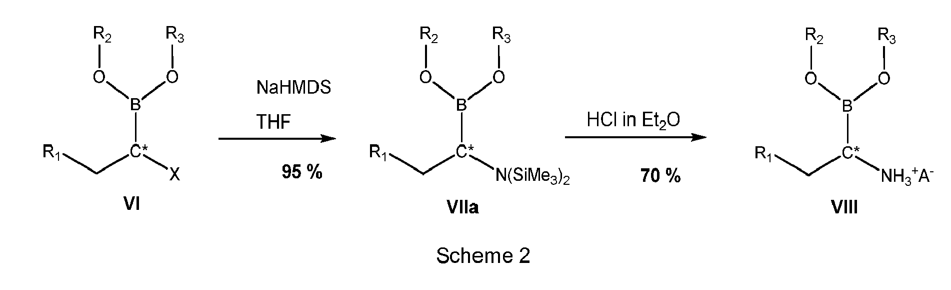

According to the preferred embodiment of Scheme 2, a compound of formula VIII, for example about 3.8 mmol, in form of its (*R*)- or (*S*)- enantiomer or in form of a mixture of enantiomers, can be prepared by contacting a compound of formula VI dissolved in an organic solvent, preferably THF, with a solution comprising a reagent for substituting X with a protected amino moiety, for example sodium bis(trimethylsilyl)amide (NaHMDS), at suitably low temperature such as -60 °C to -10 °C, more preferably -40 °C to -30 °C, in an inert, preferably argon atmosphere. The solution is warmed to room temperature such as about 20 °C to 25 °C, and stirred for a suitable period of time, for example 1 to 15 hours, preferably for about 5 hours. Then, the reaction mixture is evaporated to dryness and the residue is subsequently dissolved in a suitable volume of *n*-heptane, for example about 10 mL, washed with a suitable volume of H₂O, for example about 8 mL, and washed with a suitable volume of saturated aqueous solution of NaCl, for example about 4 mL. The organic phase is then dried over a suitable drying agent, most preferably MgSO₄, filtrated and evaporated to dryness. Compound of formula VIIa obtained in such a manner is subsequently further converted into the compound of formula VIII by dissolving the previously obtained residue in a suitable volume of *n*-heptane, for example 20 mL, and by adding a suitable amount of anhydrous acidic solution, for example anhydrous HCl solution in Et₂O, at a suitably low temperature such as -100 °C to -10 °C, more preferably -70 °C to -50 °C, in an inert, preferably argon atmosphere. The reaction mixture is warmed to room temperature, such as about 20 °C to 25 °C, and finally the precipitating solid is isolated from reaction mixture by filtration and washed with Et₂O to give α-amino boronic ester (VIII).

According to another embodiment of the present invention, the racemic mixture of the α-amino boronic ester (VIII) obtained above can be further separated to yield optically pure (*R*)- or (*S*)-enantiomer by methods known in the art, such as enantiomeric resolution by crystallization with chiral acids, e.g. malic acid, tartaric acid, mandelic acid, or by chiral chromatography. In the special case wherein the borolane part of compound of formula VIII is chiral, compound of formula VIII represents a diastereomer. Since diastereomers differ in their scalar characteristics, diastereomeric comounds of formula VIII can be separated without providing a chiral environment, e.g by crystallization or chromatographic methods on achiral supporters.

Enantiomers obtained in such manner can then be subjected to further synthesis steps to yield compounds of general formula X or free acids or esters or anhydrides or salts thereof 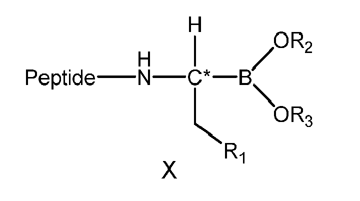 , wherein R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
peptide comprises 1-6 amino acids coupled to each other by peptide bonds with optionally acylated terminal amino group, and
wherein the chiral center * is in its (*R*) or (*S*) configuration.

For example, the racemic mixture of the intermediate compound of formula VIII, for example 3-methyl-1-(4,4,5,5-tetramethyl-1,3,2-dioxaborolan-2-yl)butan-1-amine hydrochloride is first separated by enantiomeric resolution method known in the art to give (*R*)-3-methyl-1-(4,4,5,5-tetramethyl-1,3,2-dioxaborolan-2-yl)butan-1-amine hydrochloride, which can then be subjected to further synthesis steps to yield bortezomib by synthesis routes known to or readily devisable by a person skilled in the art. For example, the following synthesis routes may be applied:

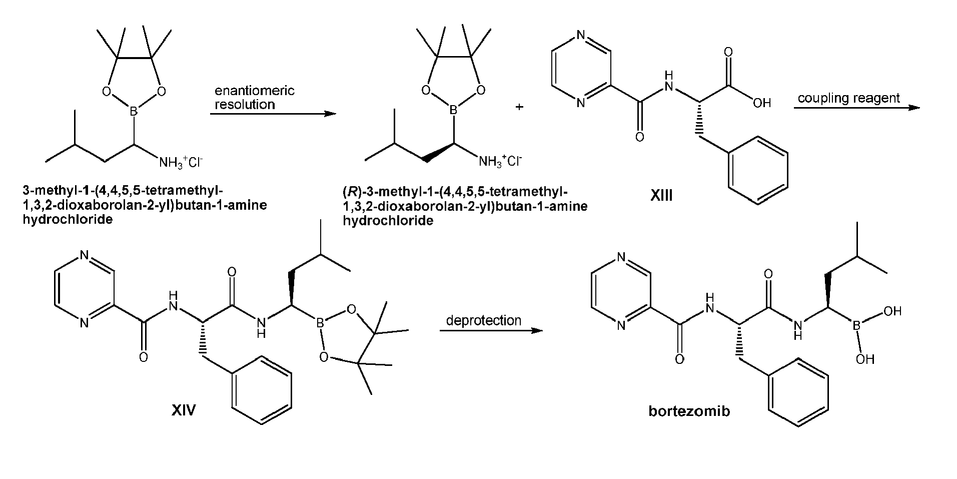

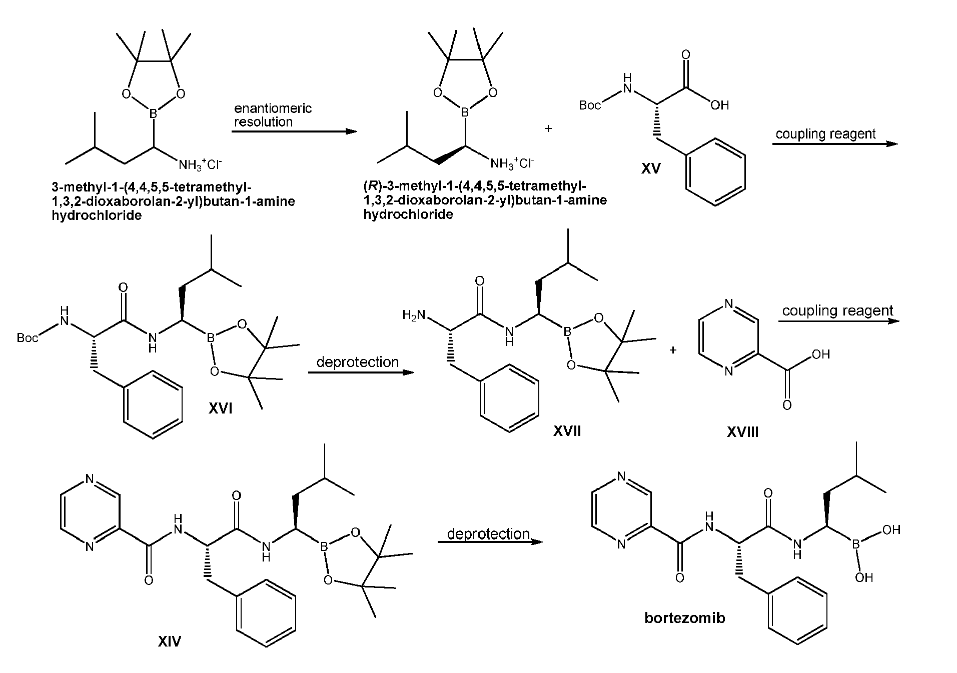

### Experimental Procedures

### Example 1:

### Synthesis of 4,4,5,5-tetramethyl-2-(3-methylbut-1-ynyl)-1,3,2-dioxaborolane (IIIa)

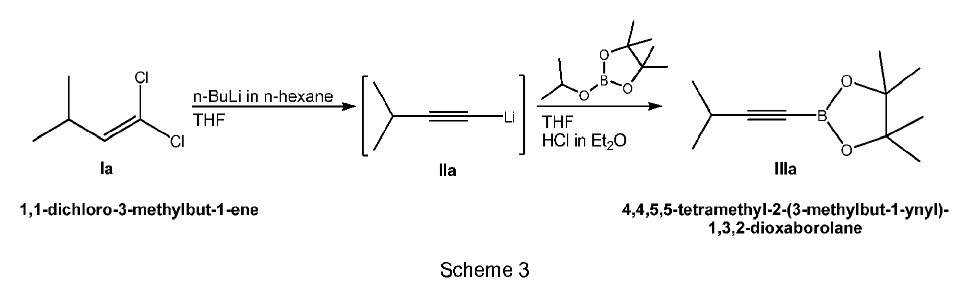

To a stirred solution of la (7.0 g, 50 mmol) in dry THF (25 mL) at -78 °C was added *n*-BuLi (1.6 M in *n-*hexane, 62.5 mL, 100 mmol). After being stirred for 1 hour at -78 °C, the reaction mixture was warmed to room temperature and stirred for 1 hour at that temperature. Another flask was charged with 2-isopropoxy-4,4,5,5-tetramethyl-1,3,2-dioxaborolane (7.8 mL, 38 mmol) in dry THF (50 mL) under argon atmosphere, and the reaction mixture was cooled to -78 °C. The lithium acetylide from the first flask, which was cooled to -78 °C, was slowly added to the second by a double-ended needle. The mixture was stirred at -78 °C for 2 hours, after which anhydrous HCl (100 mmol) was added. Then, reaction mixture was warmed to room temperature. After removal of the precipitated LiCl either by filtration and removal of solvents under reduced pressure, the residue was purified by distillation (bp = 113-115 °C/7 mbar) to afford IIIa (5.9 g, 80 %) as an oil.
¹H NMR (CDCl₃): δ (ppm) = 1.15 (d, 6H), 1.25 (s, 12H), 2.45-2.6 (m, 1H).
¹³C NMR (CDCl₃): δ (ppm) = 21.0, 22.2, 24.5, 83.9.

Starting material la was prepared as described in Tetrahedron Letters 2000, 41, 4007-4009.

### Example 2:

### Synthesis of 2-(1-chloro-3-methylbut-1-enyl)-4,4,5,5-tetramethyl-1,3,2-dioxaboro-lane (Va)

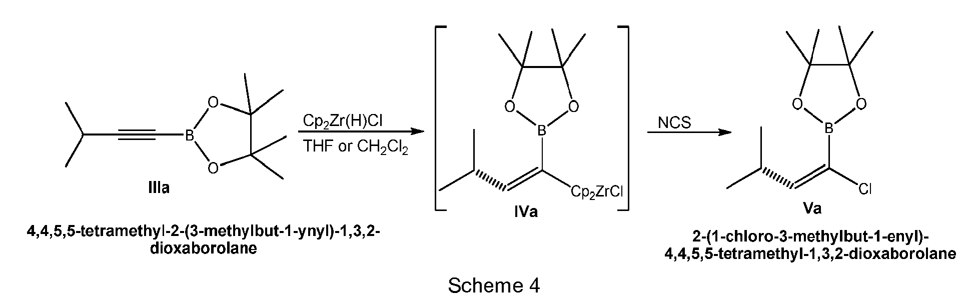

### a) With THF as a solvent

A suspension of Cp₂Zr(H)Cl (7.3 g, 27 mmol) in dry THF (55 mL) was stirred at room temperature under argon atmosphere. Then, a 48 mL of 0.5 M solution of IIIa (24 mmol) in dry THF was added. The reaction mixture was stirred for 1 hour, resulting in a clear orange solution. Addition of *N*-chlorosuccinimide (3.6 g, 27 mmol) *in situ* led to the discharge of the colour of the solution. The solvent was removed under reduced pressure, and *n*-hexane (5 x 20 mL) was added to extract the reaction mixture. After removal of solvent under reduced pressure, the residure was purified by chromatography (mobile phase: *n-*hexane/MTBE = 9.5/0.5) to afford Va (2.5 g, 45 %) as an oil.

### b) With CH₂Cl₂ as a solvent

A suspension of Cp₂Zr(H)Cl (7.3 g, 27 mmol) in dry CH₂Cl₂ (55 mL) was stirred at room temperature under argon atmosphere. Then, a 48 mL of 0.5 M solution of IIIa (24 mmol) in CH₂Cl₂ was added. The reaction mixture was stirred for 1 hour, resulting in a clear orange solution. Addition of *N-*chlorosuccinimide (3.6 g, 27 mmol) *in situ* led to the discharge of the colour of the solution. The solvent was removed under reduced pressure, and *n*-hexane (5 x 20 mL) was added to extract the reaction mixture. After removal of solvent under reduced pressure, the residure was purified by chromatography (mobile phase: *n*-hexane/MTBE = 9.5/0.5) to afford Va (3.3 g, 60 %) as an oil.
¹H NMR (CDCl₃): δ (ppm) = 0.95 (d, 6H), 1.25 (s, 12H), 2.95-3.05 (m, 1H), 6.35 (d, 1H).
¹³C NMR (CDCl₃): δ (ppm) = 22.8, 24.6, 29.5, 84.2, 156.2.

### Example 3a:

### Synthesis of 2-(1-chloro-3-methylbutyl)-4,4,5,5-tetramethyl-1,3,2-dioxaborolane (VIa)

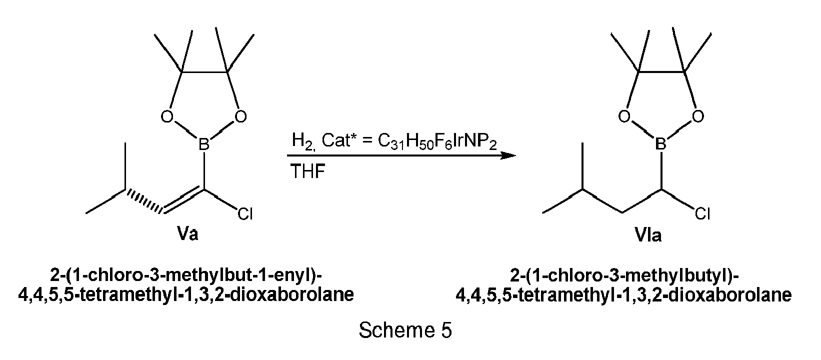

The 75 mL stainless steel autoclave was flushed with nitrogen. The product Va (1.15 g, 5.0 mmol), the (1,5-cyclo-octadiene)(pyridine)(tricyclohexylphosphine)iridium(I) hexafluorophosphate (80.5 mg, 0.1 mmol) and dry THF (30 mL) were quickly placed in the autoclave under nitrogen atmosphere. The autoclave was sealed and pressurized/depressurized first 3 times with 6 bar of nitrogen, then 3 times with 6 bar of hydrogen. The mixture was stirred for 10 days at 50 °C under 10 bar of hydrogen. Once the autoclave had cooled to room temperature, the autoclave was carefully depressurized, the solution was poured into a round bottomed flask. The solvent was removed under reduced pressure and the residue was passed through a short column of silica gel (eluent = *n*-hexane) to removed catalyst. The product VIa (0.84 g, 80 %) was carried over into next step without further purification.
¹H NMR (CDCl₃): δ (ppm) = 0.9 (m, 6H), 1.3 (s, 1H), 1.45-1.5 (m,1H), 1.75-1.85 (m, 1H), 1.87-1.95 (m, 1H), 3.5-3.6 (m, 1H).
¹³C NMR (CDCl₃): δ (ppm) = 22.8, 24.5, 25.5, 31.6, 42.5, 84.3.

### Example 3b-d:

### Synthesis of (R)- or (S)-2-(1-chloro-3-methylbutyl)-4,4,5,5-tetramethyl-1,3,2-dioxaborolane (R- or S-VIa)

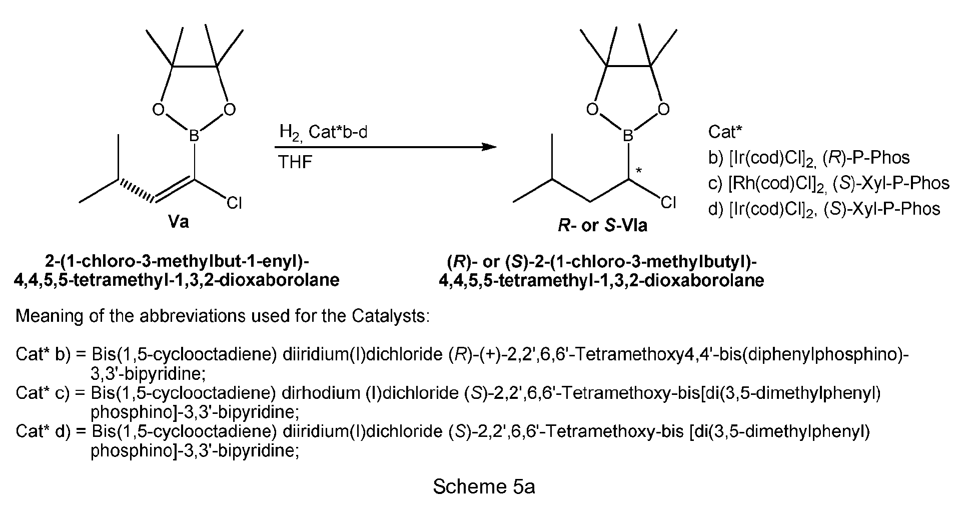

Catalyst preparation (Cat*b-d): The appropriate phosphine ligand (1.1 eq) and the metal precursor (1.0 eq) are stirred in dry THF under nitrogen atmosphere for 30 min at room temperature. The solvent is removed by evaporation and the solid is stored under nitrogen.

The 75 mL stainless steel autoclave is flushed with nitrogen. The product Va (1.15 g, 5.0 mmol), the appropriate catalyst (Cat*b-d; 80.5 mg, 0.1 mmol) and dry THF (30 mL) are quickly placed in the autoclave under nitrogen atmosphere. The autoclave is sealed and pressurized/depressurized first 3 times with 6 bar of nitrogen, then 3 times with 6 bar of hydrogen. The mixture is stirred for 10 days at 50 °C under 10 bar of hydrogen. Once the autoclave is cooled to room temperature, the autoclave is carefully depressurized, the solution is poured into a round bottomed flask. The solvent is removed under reduced pressure and the residue is passed through a short column of silica gel (eluent = *n*-hexane) to removed catalyst. The product *R*- or *S*-VIa is carried over into next step without further purification. In case the catalysis does not provide for sufficient enantiomeric excess, e.g. enantiomeric excess is less than about 99%, enantiomeric resolution can be applied prior to carrying out the next reaction step.

### Example 4:

### Synthesis of 3-methyl-1-(4,4,5,5-tetramethyl-1,3,2-dioxaborolan-2-yl)butan-1-amine hydrochloride (leucine boronate hydrochloride, VIIIa)

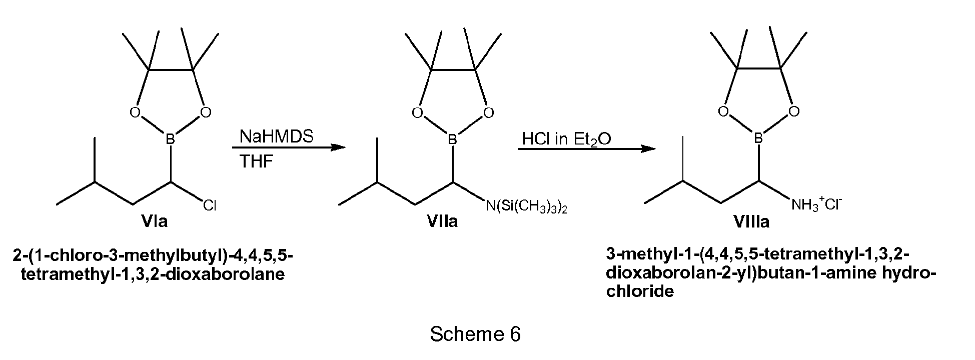

To a solution of NaHMDS (1M in THF, 3.8 mL, 3.8 mmol) in dry THF (8 mL) at -35 °C under argon atmosphere was added VIa (0.88 g, 3.8 mmol) dissolved in 8 mL dry THF. The solution was warmed to room temperature and stirred for 5 hours. The reaction mixture was evaporated to dryness. The residue was dissolved in 10 mL of *n*-heptane, washed with 8 mL H₂O and 4 mL saturated aqueous solution of NaCl. The organic phases was dried over MgSO₄, filtrated and evaporated to dryness. To a solution of the resulting residue taken up in *n*-heptane (20 mL), anhydrous HCl (4 eq, solution in Et₂O) was added at -60 °C under argon atmosphere. Then, reaction mixture was warmed to room temperature. The precipitating solid was isolated from reaction mixture by filtration and washed with Et₂O to afford VIIIa (0.63 g, 70 %) as a white solid.
¹H NMR (CDCl₃): δ (ppm) = 0.9 (d, 6H), 1.25 (s, 12 H), 1.55-1.65 (m,1H), 1.7-1.8 (m, 1H), 1.82-1.9 (m, 1H), 2.85-2.95 (m, 1H), 8.2 (s, 3H).
¹³C NMR (CDCl₃): δ (ppm) = 22.4, 22.4, 24.6, 24.9, 25.0, 38.5, 84.9.

## Claims

1. A process for preparing a compound of formula VI 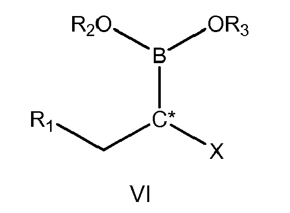 , wherein:
R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or
unfused ring; and
X is selected from the group consisting of Cl, Br, I, OCOR' and OSO₂R', wherein R' represents substituted or unsubstituted alkyl, substituted or unsubstituted aryl or substituted or unsubstituted aralkyl; and
* indicates a chiral center;
wherein said process comprises the steps of:
(i) providing a compound of formula V 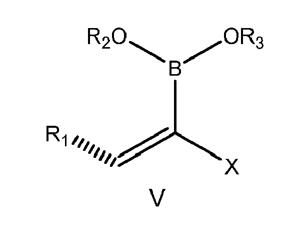
, wherein R₁, R₂, R₃ and X are as defined above;
and
(ii) converting said compound of formula V to compound of formula VI by hydrogenation, and
(iii) optionally applying enantiomeric resolution in order to obtain enantiomerically pure compound of formula VI.

2. The process according to claim 1, wherein R₁ is hydrogen or is selected from a group consisting of substituted or unsubstituted linear C₁-C₅-alkyl, substituted or unsubstituted branched C₁-C₅-alkyl and substituted or unsubstituted C₃-C₈-cycloalkyl, wherein R₂ and R₃ are selected from a group consisting of linear substituted or unsubstituted C₁-C₅-alkyl, substituted or unsubstituted branched C₁-C₅-alkyl, or R₂ and R₃ form a part of a 5- to 10-membered fused or unfused ring, optionally a chiral 5- to 10-membered fused or unfused ring; preferably, R₁ is unsubstituted linear or branched C₁-C₅-alkyl, and/or R₂ and R₃ form a part of a 5-membered ring; more preferably, R₁ is isopropyl and/or R₂ and R₃ cooperatively form a part of a 5-membered ring representing 4,4,5,5-tetramethyl-[1 ,3,2] dioxaborolane; and/or
wherein X is a halogen selected from the group consisting of Cl, Br and I.

3. The process according to claim 1 or 2, wherein said hydrogenation is conducted in the presence of a catalyst, preferably said catalyst is a catalyst for homogeneous catalysis; and/or
wherein the catalyst is selected from complexes comprising at least one transition metal; preferably wherein the ligands of said complex are selected from at least one ligand containing electron-rich species such as various double bonded compounds and/or free electron pair containing O, N, S, or P species, wherein more preferably the transition metal catalyst has chirality in the ligand and/or at the transition metal atom, or the transition metal complex having chirality is formed in situ by using an achiral procatalyst comprising the transition metal together with a cocatalyst having chirality; and/or
wherein the transition metal comprised in the complex is selected from the group consisting of Cu, Co, Ni, Rh, Ru, Pd and Ir, preferably Rh, Ru, Pd and Ir, more preferably Ru and Ir, and in particular Ir; and/or
wherein the catalyst is (1,5-cyclo-octadiene)(pyridine)(tricyclohexyl-phosphine) iridium(I) hexafluorophosphate.

4. The process according to claim 3, wherein dehalogenation is essentially avoided during hydrogenation, preferably dehalogenation occurs in less than 10 molar %, more preferably in less than 5 molar %, most preferably in less than 3 molar %, in particular in less than molar 1% relative to the molar amount of compound of formula VI.

5. The process according to any one of claims 1 to 4, wherein the compound of formula VI 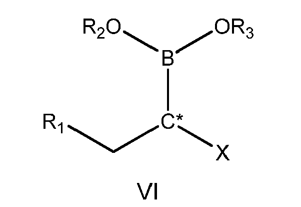 , wherein R₁, R₂, R₃ are defined above, and X is selected from the group consisting of Cl, Br, I, OCOR' and OSO₂R',
is further converted to a compound of formula VI' 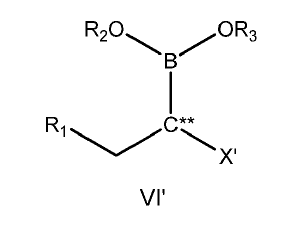 , wherein R₁, R₂, R₃ are defined above, and X' is selected from the group consisting of Cl, Br, I, OCOR' and OSO₂R',
and X' is not the same as X,
wherein a chiral center ** has the opposite stereochemical configuration of the chiral center *,
by applying a nucleophilic substitution of type S_{N}2.

6. The process according to any one of the preceding claims, wherein the compound of formula V 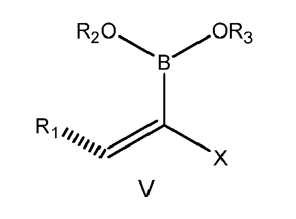 , wherein R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
X is selected from the group consisting of Cl, Br, I, OCOR' and OSO₂R', wherein R' represents substituted or unsubstituted alkyl, substituted or unsubstituted aryl or substituted or unsubstituted aralkyl,
is prepared from a compound of formula III 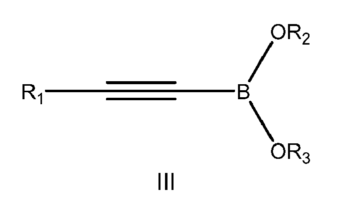 , wherein R₁, R₂ and R₃ are as defined above,
by hydrozirconation converting compound of formula III into compound of formula IV 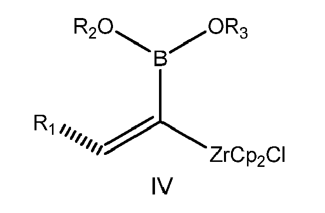 , wherein the ZrCp₂Cl moiety of compound of formula IV is substituted by X, wherein X comprises Cl, Br and I by means of a reaction of halogenation; and
optionally a halogen as defined above is further converted to compound of formula V in which X is selected from the group consisting of OCOR' and OSO₂R', wherein R' are as defined above.

7. A process for preparing a compound of formula III 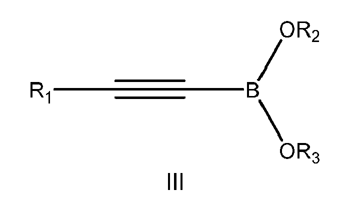 , wherein R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
wherein said process comprises the steps of:
(i) providing a compound of formula I 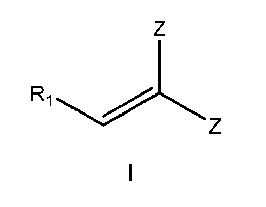 , wherein R₁ is substituted or unsubstituted alkyl, substituted or unsubstituted aryl, or substituted or unsubstituted aralkyl;
Z is selected from the group consisting of Cl, Br and I, preferably Cl,
(ii) adding a strong organometallic base to a solution of a compound of formula I in order to convert compound of formula I to compound of formula II 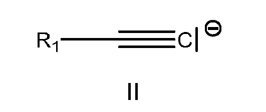 , wherein R₁ is defined as above,
(iii) reacting the compound of formula II with a compound of formula IX 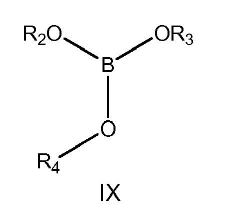 , wherein R₂ and R₃ are defined as above,
and R₄ represents substituted or unsubstituted alkyl, substituted or unsubstituted aryl, or substituted or unsubstituted aralkyl, wherein R₄ is same or different group as R₂ and/or R₃, in the presence of a solvent followed by addition of an acid,
wherein steps (i), (ii) and (iii) are performed as a one pot reaction without isolation of compound of formula II or its respective acetylene derivative R₁C≡CH.

8. The process according to claim 6, wherein the compound of formula III 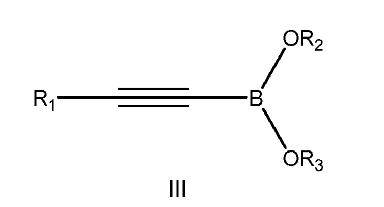 , wherein R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
is prepared from a compound of formula II 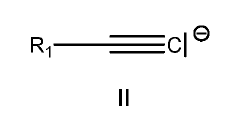 , wherein R₁ is as defined above,
by reacting the compound of formula II with a compound of formula IX 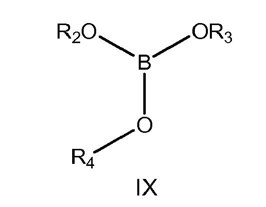 , wherein R₂ and R₃ are defined as above,
and R₄ represents substituted or unsubstituted alkyl, substituted or unsubstituted aryl, or substituted or unsubstituted aralkyl, wherein R₄ is same or different group as R₂ and/or R₃,
in the presence of a solvent followed by addition of an acid.

9. The process according to claim 7 or 8, wherein the compound of formula II is prepared from a compound of formula I 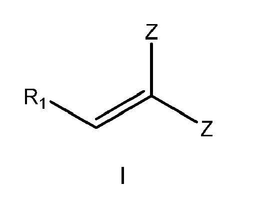 , wherein R₁ is hydrogen, substituted or unsubstituted alkyl, substituted or unsubstituted aryl, or substituted or unsubstituted aralkyl;
Z is selected from the group consisting of Cl, Br and I, preferably Cl; by adding a strong organometallic base to a solution of a compound of formula I,
preferably said strong organometallic base is selected from the group consisting of metal amides and alkyl lithiums, preferably sodium amide, potassium amide, lithium amide, lithium diisopropylamide, methyllithium, butyllithium, hexyllithium and phenyllithium, more preferably butyllithium (BuLi) or methyllithium, even more preferably BuLi, in particular n-BuLi.

10. The process according to any one of claims 7 to 9, wherein R₂ and/or R₃ of compound of formula IX is/are chiral, preferably a borolane ring formed by R₂ and R₃ of compound of formula IX is chiral, more preferably said chiral compounds of formula IX are enantiomerically or diastereomerically pure.

11. The process according to any one of claims 1 to 6, wherein the compound of formula VI 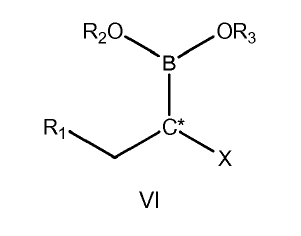 , wherein R₁, R₂ and R₃ are defined as above, and X is selected from the group consisting of Cl, Br I, OCOR' and OSO₂R', and
* indicates a chiral center;
is further converted to a compound of formula VIII 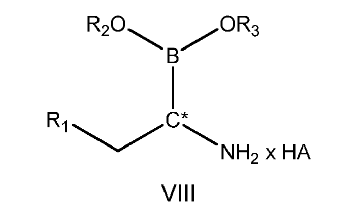 , wherein R₁, R₂ and R₃ are as defined above; and
A is an anion selected from the group of anions consisting of Cl⁻, Br⁻, HSO₄⁻, CH₃COO⁻, CF₃COO⁻ and R'SO₃⁻, wherein R' represents alkyl or aryl; and
* indicates a chiral center;
by a process comprising the steps of:
(i) converting the compound of formula VI to compound of formula VII 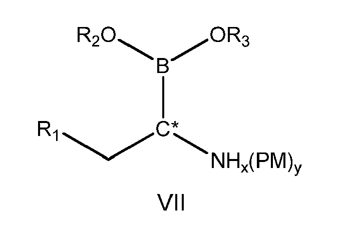 , wherein R₁, R₂ and R₃ are as defined above, and
PM is an amino group protecting moiety, wherein PM is selected from the group consisting of *tert-*butanesulfinyl (SO-*t*-Bu), tosyl (Ts), *p*-nitrobenzenesulfonyl (Ns), carbobenzyloxy (Cbz), *t-*butyloxycarbonyl (Boc), benzyl (Bn), *p*-methoxybenzyl (MPM), dimethoxybenzyl (Dmb), *p-*hydroxybenzyl (HBn), 9-phenylfluoren-9-yl (Pf), fluorenyl (Flu), diphenylmethyl (DPM), ferrocenylmethyl (Fcm) and 4-Methyltrityl (Mtt), and x = 1 and y = 1, or PM is SiR"₃, wherein R" represents alkyl and x = 0 and y = 2; and
* indicates a chiral center;
by substituting X of the compound of formula VI with a protected amino group,
and
(ii) subjecting the compound of formula VII to cleavage of protection groups to yield the compound of formula VIII
and
(iii) optionally applying enantiomeric resolution in order to obtain enantiomerically pure compound of formula VIII.

12. A compound of formula IV 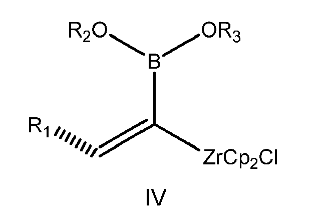 , wherein
R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
with the proviso that R₁ does not denote *t*-Bu or n-Bu.

13. A compound of formula V 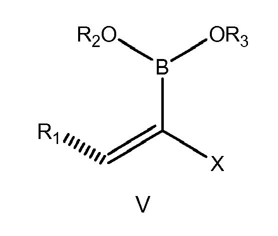 , wherein
R₁ is hydrogen, substituted or unsubstituted alkyl, substituted or unsubstituted aryl, or substituted or unsubstituted aralkyl;
R₂ and R₃ independently from each oter represent substituted or unsubstituted alkyl, substituted or unsubstituted aryl, or substituted or unsubstituted aralkyl, or R₂ and R₃ cooperatively form a part of a 5- to 10-membered fused or unfused ring; and X is selected from the group consisting of Cl, Br, I, OOCR' and OSO₂R', wherein R' represents alkyl or aryl;
with the proviso that if X is Cl, Br, I, then R₁ does not denote *t*-Bu.

14. A compound of formula IVa

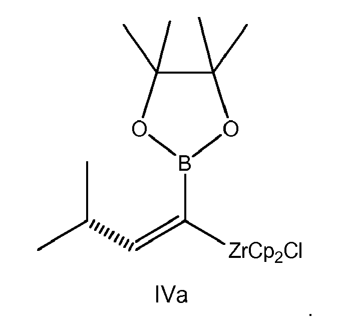

15. A compound of formula Va 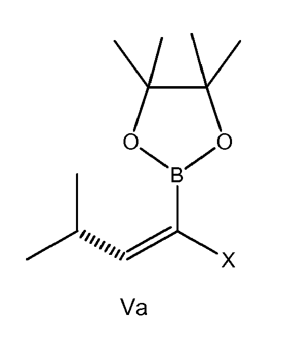 , wherein X is selected from the group consisting of Cl, Br, I, OCOR' and OSO₂R', wherein R' represents alkyl or aryl.

16. A process for producing *N*-terminal peptidyl boronic acid derivatives of formula X 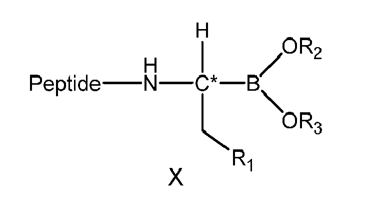 , wherein R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and
peptide comprises 1-6 amino acids coupled to each other by peptide bonds with optionally acylated terminal amino group; and
the chiral center * is in its (*R*) or (*S*) configuration;
or free acid or ester or anhydride or salt thereof, comprising the steps of:
(i) providing a compound of formula VI prepared by a process according to any one of claims 1 to 6,
(ii) converting said compound of formula VI to compound of formula VIII by a process according to claim 11,
(iii) optionally obtaining enantiomerically pure compound of formula VIII by enantiomeric resolution, and
(iv) converting said compound of formula VIII to *N*-terminal peptidyl boronic acid derivative of formula X or free acid or ester or anhydride or salt thereof.

17. A process for producing bortezomib (*N*-(pyrazin-2-yl)carbonyl-L-phenylalanine-L-leucine boronic acid) or ester or anhydride or salt thereof, comprising the steps of:
(i) providing a compound of formula VI prepared by a process according to any one of claims 1 to 6,
(ii) converting said compound of formula VI to compound of formula VIII by a process according to claim 11,
(iii) optionally obtaining enantiomerically pure compound of formula VIII by enantiomeric resolution, and
(iv) converting said compound of formula VIII to bortezomib or ester or anhydride or salt thereof.

18. A process for producing a pharmaceutical composition comprising the steps of:
(i) preparing *N*-terminal peptidyl boronic acid derivative of formula X or free acid or ester or anhydride or salt thereof according to claim 16, and
(ii) mixing said *N*-terminal peptidyl boronic acid derivative of formula X or free acid or ester or anhydride or salt thereof with at least one excipient.

19. A process for producing a pharmaceutical composition comprising the steps of:
(i) preparing bortezomib (*N*-(pyrazin-2-yl)carbonyl-L-phenylalanine-L-leucine boronic acid) or ester or anhydride or salt thereof according to claim 17, and
(ii) mixing said bortezomib with at least one excipient.

20. Use of a compound of formula V 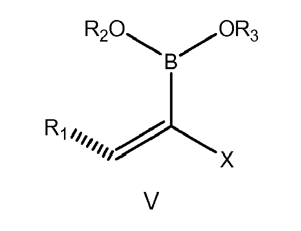 , wherein
R₁ is hydrogen, substituted or unsubstituted alkyl, substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring; and X is selected from the group consisting of Cl, Br, I, OCOR' or OSO₂R' wherein R' represents alkyl or aryl;
in the process for preparation of compound of formula VIII 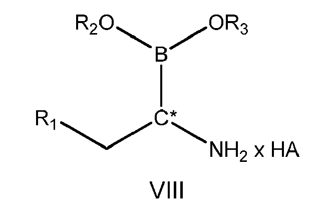 , wherein R₁, R₂ and R₃ are as defined above; and
A is an anion selected from the group of anions consisting of Cl⁻, Br⁻, HSO₄, CH₃COO⁻, CF₃COO⁻ and R'SO₃⁻, wherein R' represents alkyl or aryl; and
* indicates a chiral center; or free amine thereof
and/or *N*-terminal peptidyl boronic acid derivative of formula X or free acid or ester or anhydride or a pharmaceutically acceptable salt thereof, preferably bortezomib or ester or anhydride or a pharmaceutically acceptable salt thereof.

21. Use of a compound of formula IVa 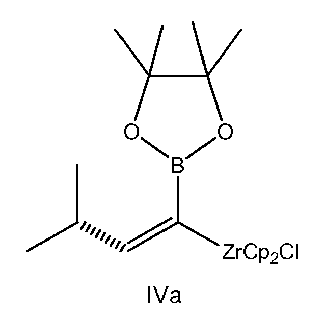 in the process for preparation of compound of formula VIIIa 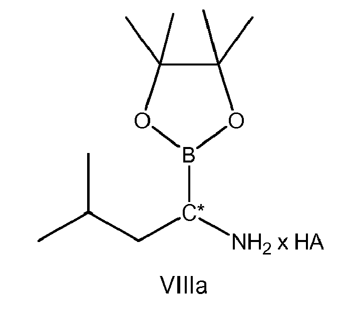 , wherein A is an anion selected from the group of anions consisting of Cl⁻, Br⁻, HSO₄, CH₃COO⁻, CF₃COO⁻ and R'SO₃⁻, wherein R' represents alkyl or aryl;
* indicates a chiral center; or free amine thereof,
and/or *N*-terminal peptidyl boronic acid derivative of formula X or free acid or ester or anhydride or a pharmaceutically acceptable salt thereof, preferably bortezomib or ester or anhydride or a pharmaceutically acceptable salt thereof.

22. Use of a compound of formula Va 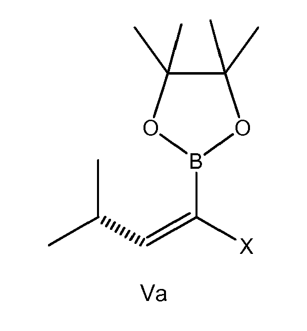 , wherein X is selected from the group consisting of Cl, Br, I, OCOR' or OSO₂R' wherein R' represents alkyl or aryl; preferably X is selected from the group consisting of Cl, Br and I
in the process for preparation of compound of formula VIIIa 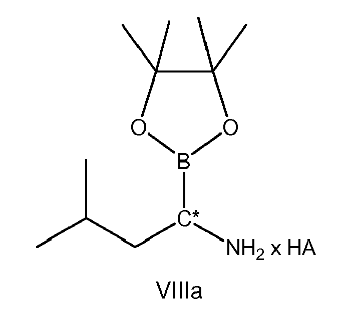 , wherein A is an anion selected from the group of anions consisting of Cl⁻, Br⁻, HSO₄, CH₃COO⁻, CF₃COO⁻ and R'SO₃⁻, wherein R' represents alkyl or aryl;
* indicates a chiral center; or free amine thereof,
and/or *N*-terminal peptidyl boronic acid derivative of formula X or free acid or ester or anhydride or a pharmaceutically acceptable salt thereof, preferably bortezomib or ester or anhydride or a pharmaceutically acceptable salt thereof.

23. Use of an iridium catalyst for hydrogenation of compounds of formula XI where dehalogenation occurs in less than 10 molar % relative to the molar amount of hydrogenated, non-dehalogenated product obtained from the compound of formula XI: 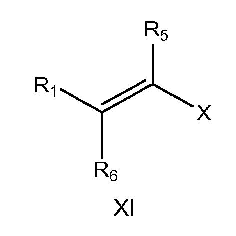 , wherein
R₁ and R₆ is hydrogen, substituted or unsubstituted alkyl substituted or unsubstituted aryl, or substituted or unsubstituted aralkyl;
R₅ is OR', NR'R", SR', X", B(OR')₂, in which X" is selected from F, Cl, Br, I, OCOR', OSO₂R' and R' is substituted or unsubstituted alkyl substituted or unsubstituted aryl, or substituted or unsubstituted aralkyl;
X is selected from Cl, Br, I.

24. Use of an iridium catalyst for hydrogenation of compounds of formula XII where dehalogenation occurs in less than 10 molar % relative to the molar amount of hydrogenated, non-dehalogenated product obtained from the compound of formula XII: 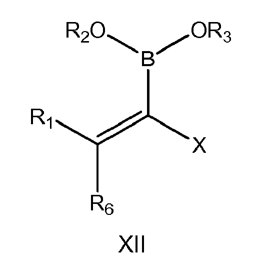 , wherein
R₁ and R₆ is hydrogen, substituted or unsubstituted alkyl substituted or unsubstituted aryl, or substituted or unsubstituted aralkyl;
R₂ and R₃ independently from each other represent substituted or unsubstituted alkyl, substituted or unsubstituted aryl, or substituted or unsubstituted aralkyl, or R₂ and R₃ cooperatively form a part of a 5- to 10-membered fused or unfused ring, optionally a chiral 5- to 10-membered fused or unfused ring;
X is Cl, Br, I.
